(19) Europäisches Patentamt
European Patent Office
Office européen des brevets

(11) EP 2 886 593 A1

(12) EUROPÄISCHE PATENTANMELDUNG

(43) Veröffentlichungstag:
**24.06.2015 Patentblatt 2015/26**

(51) Int Cl.:
***C08K 5/00*** *(2006.01)* ***C08G 59/42*** *(2006.01)*
***C07C 51/00*** *(2006.01)* ***C07D 307/78*** *(2006.01)*

(21) Anmeldenummer: **14196246.4**

(22) Anmeldetag: **04.12.2014**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **19.12.2013 DE 102013226613**

(71) Anmelder: **Evonik Industries AG**
**45128 Essen (DE)**

(72) Erfinder:
- **Willy, Benjamin**
**40211 Düsseldorf (DE)**
- **Neumann, Manfred**
**45770 Marl (DE)**

# (54) Verarbeitungsfreundlicher Dianhydridhärter für Epoxidharzsysteme basierend auf 5,5'-Oxybis(isobenzofuran-1,3-dion)

(57) Die vorliegende Erfindung betrifft eine Zusammensetzung umfassend 5,5'-Oxybis(isobenzofuran-1,3-dion), 4,4'-Oxybis(*ortho*-phthalsäure) und mindestens eine Monoanhydridverbindung ausgewählt aus der Gruppe bestehend aus Methylhexahydroisobenzofuran-1,3-dion, 5-Methyl-3a,4,7,7a-tetrahydro-4,7-methanoisobenzofuran-1,3-dion, 5-Methyl-3a,4,7,7a-tetrahydroisobenzofuran-1,3-dion, 3-Methylfuran-2,5-dion, 3,3,4,4,5,5-Hexafluorodihydro-2*H*-pyran-2,6(3*H*)-dion, 3,3-Dimethyldihydrofuran-2,5-dion. Die Erfindung betrifft auch ein Härtersystem für Epoxidharze, welches diese Zusammensetzung umfasst. Die Erfindung betrifft auch die Verwendung des Härtersystems zur Härtung von Epoxidharzen sowie entsprechende Verfahren.

EP 2 886 593 A1

## Beschreibung

Die vorliegende Erfindung betrifft eine Zusammensetzung umfassend

a. 5,5'-Oxybis(isobenzofuran-1,3-dion);
b. 4,4'-Oxybis(*ortho*-phthalsäure); und
c. mindestens eine Monoanhydridverbindung ausgewählt aus der Gruppe bestehend aus Methylhexahydroisobenzofuran-1,3-dion, 5-Methyl-3a,4,7,7a-tetrahydro-4,7-methanoisobenzofuran-1,3-dion, 5-Methyl-3a,4,7,7a-tetrahydroisobenzofuran-1,3-dion, 3-Methylfuran-2,5-dion, 3,3,4,4,5,5-Hexafluorodihydro-2*H*-pyran-2,6(3*H*)-dion, 3,3-Dimethyldihydrofuran-2,5-dion.

Die Erfindung betrifft auch ein Härtersystem für Epoxidharze, welches diese Zusammensetzung umfasst. Die Erfindung betrifft auch die Verwendung des Härtersystems zur Härtung von Epoxidharzen sowie entsprechende Verfahren.

## Hintergrund der Erfindung

Epoxidharze gehören zu den vielseitigsten polymeren Werkstoffen. Sie finden Anwendungen z. B. als Beschichtungen, Klebstoffe, Gießharzmassen, Formmassen, als Einbettmassen zur Umhüllung elektronischer Bauteile, als Laminate und Basismaterial für gedruckte Schaltungen sowie als Matrixharze für faserverstärkte Kunststoffe.

Die Überführung von monomeren oder oligomeren Epoxidharzen in polymere Stoffe erfordert Reaktionspartner, die man als Härter oder Härtungsmittel bezeichnet. Je nach Typ des Härters erfolgt die Härtungsreaktion bei Temperaturen um Raumtemperatur oder niedrigen Temperaturen (sogenannte "Kalthärtung") oder bei erhöhten Temperaturen (sogenannte "Warm- oder Heißhärtung"). Zur Härtung von Epoxidharzen bei niedrigen Temperaturen für technische Anwendungen werden vorwiegend nur aliphatische primäre oder sekundäre Amine und Polyamine eingesetzt, seltener dagegen Polythiole oder spezielle Salze.

Alle unmodifizierten Amine reagieren alkalisch bis stark alkalisch. Flüssige Amine, insbesondere die aliphatischen und cycloaliphatischen, können Hautschädigungen bis zur Verätzung verursachen. Nachteilig ist auch die hohe Flüchtigkeit der flüssigen Amine. Ein großer Nachteil der Kalthärtung von Epoxidharzen mit den oben erwähnten Härtungsmitteln ist die geringe Temperatur- und Chemikalienbeständigkeit der entstehenden Produkte. Zur Erhöhung der Temperatur-, Lösungsmittel- und Chemikalienbeständigkeit ist man gezwungen, Epoxidharze bei erhöhten Temperaturen in einer Heißhärtung mit aromatischen oder cycloaliphatischen Aminen, Carbonsäureanhydriden, Polyphenolen oder mit latenten Härtern auszuhärten.

Es besteht jedoch die Nachfrage nach Epoxidharz-Härter-Systemen, die bei möglichst niedriger Temperatur aushärten können und Produkte ergeben, die eine erhöhte Temperatur-, Chemikalien- und Lösungsmittelbeständigkeit aufweisen. Potentielle Anwendungen hierfür sind z. B. Klebstoffe, Matrixharze für Faserverbundwerkstoffe und Reparaturharze für Bauteile, bei denen sich die Anwendung hoher Temperaturen nicht anbietet. Weitere Anwendungen sind Gießharz- und Einbettmassen, speziell zur Umhüllung von großen elektronischen Bauteilen, bei denen die Aushärtung bei niedriger Temperatur, mit niedriger Exothermie und folglich mit beträchtlicher Energieeinsparung ablaufen kann, wobei ein weiterer Vorteil ist, dass Produkte mit verminderter innerer Spannung entstehen.

Aus der Literatur ist bekannt, dass die Härtung von Epoxidharzen, insbesondere bei Bisphenol-A-Harzen, mit cyclischen Dicarbonsäureanhydriden und Tetracarbonsäurebisanhydriden immer Härtungstemperaturen von mindestens 120 - 150 °C erfordern, wobei dann noch Härtungszeiten von mehreren Stunden benötigt werden, siehe Houben-Weyl, Methoden der Organischen Chemie, Band E20, Makromolekulare Stoffe, Georg Thieme Verlag Stuttgart, 1987, Seite 1959. Selbst bei diesen Temperaturen ist die Vernetzungsreaktion noch so langsam, dass auf den Einsatz von Beschleunigern in der Regel nicht verzichtet werden kann. Von Vorteil ist jedoch, dass die Härtung mit Anhydriden im Vergleich zur Härtung mit Aminen mit geringerer Exothermie abläuft. Die gehärteten Produkte haben gute elektrische Isoliereigenschaften und eine gute thermische Beständigkeit.

In der Literatur ist nur ein Beispiel für die Härtung von Epoxidharzen mit cyclischen Säureanhydriden bei niedrigen Temperaturen beschrieben worden, siehe US 4,002,599. Es werden jedoch nur Systeme auf Basis polyglycidylsubstituierter Aminophenole beschrieben.

Die DE 2837726 beschreibt Epoxidharzzusammensetzungen aus mindestens einem Epoxidharz und einem Härtungsmittel, wobei das Härtungsmittel 2,3,3',4'-Diphenyltetracarbonsäureanhydrid enthält. Gemäß der Lehre der DE 2837726 muss das Dianhydrid erst gelöst werden bevor eine Härtung erfolgen kann, zum Teil wird die Mischung sogar wieder abgekühlt. Dies kann zu Problemen führen, insbesondere kann ein Abscheiden des Härtungsmittels erfolgen.

Von zusätzlichem Nachteil ist die im Stand der Technik beschriebene Notwendigkeit, einen Katalysator zur Aushärtung zu nutzen und deshalb vor allem auf den Einsatz von Aminen angewiesen zu sein. Die oben diskutierte ätzende Wirkung der aliphatischen und cycloaliphatischen Amine kann zwar etwas durch Einsatz aromatischer Amine

als Katalysatoren herabgesetzt werden, so wie es in der US 3,989,573 beschrieben ist, wo 2-Ethyl-4-methyl-imidazol eingesetzt wurde. Noch wünschenswerter wäre es allerdings, auf den Einsatz solcher Katalysatoren vollständig verzichten zu können und dennoch solche Polymerisierungsgeschwindigkeiten erhalten zu können, die den Einsatz der Härtersysteme ermöglichen.

Dazu kommt die Notwendigkeit, ein handhabbares Härtersystem zur Verfügung zu haben. So besitzen viele im Stand der Technik beschriebenen Härtersysteme, welche sich aus einer Dianhydridverbindung und einer Monoanhydridverbindung zusammensetzen, die problematische Eigenschaft, über einen weiten Mischungsbereich der beiden Komponenten als feinstaubendes Pulver vorzuliegen, was eine Verarbeitung erschwert und den Zusatz von Lösungsmitteln unumgänglich macht. Ein solcher Zusatz von Lösungsmitteln verhindert es dann wiederum, das gewünschte Härtersystem in einer hohen Konzentration, also mit möglichst wenigen anderen Stoffen, zu konfektionieren. Zum Einsatz als Härtersystem sind jedoch gerade solche hohen Konzentrationen, die allerdings nicht als Pulver vorliegen sollen, gewünscht. Des Weiteren ist es von Nachteil, wenn der Feststoff zu verdünnt vorliegt, da dann innerhalb weniger Stunden eine Absetzung des Feststoffs einsetzt, was zu unerwünschten Inhomogenitäten der Zusammensetzung führt.

Gewünscht ist daher weiterhin, eine nicht staubende Formulierung zu erhalten, die über einen weiten Mischungsbereich stabil und lagerfähig ist.

Aufgabe der vorliegenden Erfindung ist somit die Bereitstellung eines verbesserten Härtersystems für die Härtung von Epoxidharzen. Dieses Härtersystem soll einfach zu verarbeiten sein, und nach Härtung zu Harzsystemen - auch ohne den Einsatz der herkömmlichen genannten Katalysatoren - mit einer guten, dauerhaften WärmeFormbeständigkeit führen. Gleichzeitig soll das System über einen weiten Konzentrationsbereich der Dianhydridverbindung und Monoanhydridverbindung eine gute Konfektionierbarkeit erlauben und dabei bezüglich der Homogenität lagerfähig sein. Schließlich soll auch der Einsatz von Katalysatoren aus Aminverbindungen oder Metallsalzen vermieden werden und dennoch eine gute Polymerisationsgeschwindigkeit erreichbar sein.

Es wurde nun überraschend gefunden, dass diese Aufgabe gelöst wird, in dem man dem Härter 4,4'-Oxybis(ortho-phthalsäure) (im Folgenden "OTA") zur Beschleunigung der Polymerisation zusetzt.

## Kurzbeschreibung der Erfindung

Ein erster Gegenstand der vorliegenden Erfindung ist

1. eine Zusammensetzung umfassend

   a. 5,5'-Oxybis(isobenzofuran-1,3-dion) (im Folgenden "OPDA");
   b. 4,4'-Oxybis(*ortho*-phthalsäure); und
   c. mindestens eine Monoanhydridverbindung ausgewählt aus der Gruppe bestehend aus Methylhexahydroisobenzofuran-1,3-dion (im Folgenden "MHHPSA"), 5-Methyl-3a,4,7,7a-tetrahydro-4,7-methanoisobenzofuran-1,3-dion (im Folgenden "MNA"), 5-Methyl-3a,4,7,7a-tetrahydroisobenzofuran-1,3-dion (im Folgenden "MTHPA"), 3-Methylfuran-2,5-dion (im Folgenden "MFD"), 3,3,4,4,5,5-Hexafluorodihydro-2*H*-pyran-2,6(3*H*)-dion (im Folgenden "HFDPA"), 3,3-Dimethyldihydrofuran-2,5-dion (im Folgenden "DMDF").

2. In einer weiteren Ausführungsform der vorliegenden Erfindung ist die Zusammensetzung nach Punkt 1 dadurch gekennzeichnet, dass die Monoanhydridverbindung ausgewählt ist aus der Gruppe bestehend aus MHHPSA, MTHPA, MNA; bevorzugt ausgewählt ist aus der Gruppe bestehend aus MTHPA, MNA.

3. In einer weiteren Ausführungsform der vorliegenden Erfindung ist die Zusammensetzung nach Punkt 1 oder 2 dadurch gekennzeichnet, dass die Monoanhydridverbindung MTHPA ist.

4. In einer weiteren Ausführungsform der vorliegenden Erfindung ist die Zusammensetzung nach einem oder mehreren der Punkte 1 bis 3 dadurch gekennzeichnet, dass das Verhältnis der Masse von OPDA in der Zusammensetzung zur Summe aus der Masse des MHHPSA, der Masse des MNA, der Masse des MTHPA, der Masse des MFD, der Masse des HFDPA, der Masse des DMDF in der Zusammensetzung 1: 0.250 bis 1: 1.900, bevorzugt 1 : 0.309 bis 1 : 1.856, bevorzugter 1 : 0.412 bis 1 : 1.546 beträgt.

5. In einer weiteren Ausführungsform der vorliegenden Erfindung ist die Zusammensetzung nach einem oder mehreren der Punkte 1 bis 4 dadurch gekennzeichnet, dass die Masse des OTA in der Zusammensetzung 0.01 bis 16.9 % der Summe aus der Masse des OPDA, der Masse des MHHPSA, der Masse des MNA, der Masse des MTHPA, der Masse des MFD, der Masse des HFDPA, der Masse des DMDF in der Zusammensetzung beträgt.

6. In einer weiteren Ausführungsform betrifft die vorliegende Erfindung ein Härtersystem für Epoxidharze, umfassend

die Zusammensetzung nach einem oder mehreren der Punkte 1 bis 5 mit einem Massenanteil von 0.10 bis 1.0, bezogen auf die gesamte Masse des Härtersystems.

7. In einer weiteren Ausführungsform der vorliegenden Erfindung ist das Härtersystem für Epoxidharze nach Punkt 6 dadurch gekennzeichnet, dass es keine Aminverbindung aufweist.

8. In einer weiteren Ausführungsform der vorliegenden Erfindung ist das Härtersystem für Epoxidharze nach Punkt 6 oder 7 dadurch gekennzeichnet, dass es kein Metallsalz aufweist.

9. In einer weiteren Ausführungsform betrifft die vorliegende Erfindung die Verwendung eines Härtersystems gemäß einem oder mehreren der Punkte 6 bis 8 zur Härtung von Epoxidharzen.

10. In einer weiteren Ausführungsform betrifft die vorliegende Erfindung ein Epoxidharzsystem umfassend ein Epoxidharz und mindestens ein Härtersystem gemäß einem oder mehreren der Punkte 6 bis 8.

Ein zweiter Gegenstand der vorliegenden Erfindung ist

11. eine Zusammensetzung bestehend aus

a. OPDA;
b. OTA; und
c. mindestens einer Monoanhydridverbindung ausgewählt aus der Gruppe bestehend aus MHHPSA, MNA, MTHPA, MFD, HFDPA, DMDF.

12. In einer weiteren Ausführungsform der vorliegenden Erfindung ist die Zusammensetzung nach Punkt 11 dadurch gekennzeichnet, dass die Monoanhydridverbindung ausgewählt ist aus der Gruppe bestehend aus MHHPSA, MTHPA, MNA; bevorzugt ausgewählt ist aus der Gruppe bestehend aus MTHPA, MNA.

13. In einer weiteren Ausführungsform der vorliegenden Erfindung ist die Zusammensetzung nach Punkt 11 oder 12 dadurch gekennzeichnet, dass die Monoanhydridverbindung MTHPA ist.

14. In einer weiteren Ausführungsform der vorliegenden Erfindung ist die Zusammensetzung nach einem oder mehreren der Punkte 11 bis 13 dadurch gekennzeichnet, dass das Verhältnis der Masse von OPDA in der Zusammensetzung zur Summe aus der Masse des MHHPSA, der Masse des MNA, der Masse des MTHPA, der Masse des MFD, der Masse des HFDPA, der Masse des DMDF in der Zusammensetzung 1: 0.250 bis 1: 1.900, bevorzugt 1 : 0.309 bis 1 : 1.856, bevorzugter 1 : 0.412 bis 1 : 1.546 beträgt.

15. In einer weiteren Ausführungsform der vorliegenden Erfindung ist die Zusammensetzung nach einem oder mehreren der Punkte 11 bis 14 dadurch gekennzeichnet, dass die Masse des OTA in der Zusammensetzung 0.01 bis 16.9 % der Summe aus der Masse des OPDA, der Masse des MHHPSA, der Masse des MNA, der Masse des MTHPA, der Masse des MFD, der Masse des HFDPA, der Masse des DMDF in der Zusammensetzung beträgt.

16. In einer weiteren Ausführungsform betrifft die vorliegende Erfindung ein Härtersystem für Epoxidharze, umfassend die Zusammensetzung nach einem oder mehreren der Punkte 11 bis 15 mit einem Massenanteil von 0.10 bis 1.0, bezogen auf die gesamte Masse des Härtersystems.

17. In einer weiteren Ausführungsform der vorliegenden Erfindung ist das Härtersystem für Epoxidharze nach Punkt 16 dadurch gekennzeichnet, dass es keine Aminverbindung aufweist.

18. In einer weiteren Ausführungsform der vorliegenden Erfindung ist das Härtersystem für Epoxidharze nach Punkt 16 oder 17 dadurch gekennzeichnet, dass es kein Metallsalz aufweist.

19. In einer weiteren Ausführungsform betrifft die vorliegende Erfindung die Verwendung eines Härtersystems gemäß einem oder mehreren der Punkte 16 bis 18 zur Härtung von Epoxidharzen.

20. In einer weiteren Ausführungsform betrifft die vorliegende Erfindung ein Epoxidharzsystem umfassend ein Epoxidharz und mindestens ein Härtersystem gemäß einem oder mehreren der Punkte 16 bis 18.

21. Ein dritter Gegenstand der vorliegenden Erfindung ist ein Verfahren zur Härtung von Epoxidharzen, wobei

a. in einem ersten Schritt mindestens ein Epoxidharz mit

i. OPDA; und
ii. mindestens einer Monoanhydridverbindung ausgewählt aus der Gruppe bestehend aus MHHPSA, MNA, MTHPA, MFD, HFDPA, DMDF

gemischt wird;

b. in einem zweiten Schritt OTA zugegeben wird; und

c. in einem dritten Schritt das Epoxidharz bei einer Temperatur von mindestens 25 °C ausgehärtet wird.

22. Ein vierter Gegenstand der vorliegenden Erfindung ist ein Verfahren zur Härtung von Epoxidharzen, wobei

a. in einem ersten Schritt

i. OPDA; und
ii. OTA; und
iii. mindestens eine Monoanhydridverbindung ausgewählt aus der Gruppe bestehend aus MHHPSA, MNA, MTHPA, MFD, HFDPA, DMDF

gemischt werden;

b. in einem zweiten Schritt mindestens ein Epoxidharz zugegeben wird; und

c. in einem dritten Schritt das Epoxidharz bei einer Temperatur von mindestens 25 °C ausgehärtet wird.

23. In einer weiteren Ausführungsform der vorliegenden Erfindung ist das Verfahren nach Punkt 21 oder 22 dadurch gekennzeichnet, dass die Aushärtung des Epoxidharzes in Schritt c. bei einer Temperatur im Bereich von 25°C bis unterhalb der Schmelztemperatur des OPDA erfolgt, die im Bereich 225 °C bis 229 °C liegt.

**Detaillierte Beschreibung der Erfindung**

Die vorliegende Erfindung betrifft demnach eine Zusammensetzung umfassend

a. OPDA;
b. OTA;
c. mindestens eine Monoanhydridverbindung ausgewählt aus der Gruppe bestehend aus MHHPSA, MNA, MTHPA, MFD, HFDPA, DMDF.

Insbesondere ist die Monoanhydridverbindung in der erfindungsgemäßen Zusammensetzung ausgewählt aus der Gruppe bestehend aus MHHPSA, MTHPA, MNA. Bevorzugter ist die Monoanhydridverbindung in der erfindungsgemäßen Zusammensetzung ausgewählt aus der Gruppe bestehend aus MTHPA, MNA. Noch bevorzugter ist die Monoanhydridverbindung in der erfindungsgemäßen Zusammensetzung MTHPA.

Die Strukturformeln der jeweiligen Verbindungen sind wie folgt.

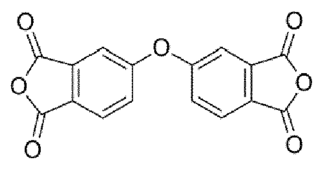
OPDA

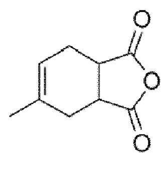
MTHPA

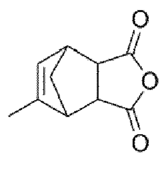
MNA

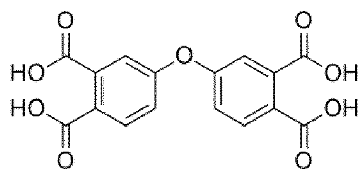
OTA

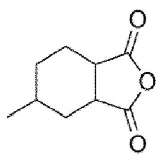
MHHPSA

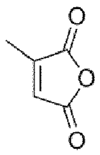
MFD

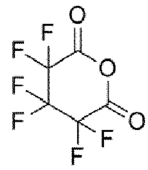
HFDPA

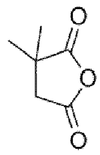
DMDF

Wie aus den Beispielen ersichtlich, zeichnet sich die erfindungsgemäße Zusammensetzung dadurch aus, dass sie aufgrund des Vorliegens des OTA schneller polymerisiert und somit auch als Härter für Epoxidharze eingesetzt werden kann. Dadurch kann auf den Einsatz stark ätzender Aminverbindungen als Katalysatoren verzichtet werden. Genauso erlaubt dies auch den Verzicht auf Metallsalze zur Katalyse, was gerade in großtechnischen Anlagen von Vorteil ist, da sich Metallsalze in diesen ablagern und typischerweise zur Korrosion von Anlagenteilen führen.

In einer vorteilhaften Ausführungsform der vorliegenden Erfindung beträgt das Verhältnis der Masse von OPDA in der erfindungsgemäßen Zusammensetzung zur Summe aus der Masse des MHHPSA, der Masse des MNA, der Masse des MTHPA, der Masse des MFD, der Masse des HFDPA, der Masse des DMDF in der erfindungsgemäßen Zusammensetzung 1 : 0.250 bis 1: 1.900, bevorzugt 1 : 0.309 bis 1 : 1.856, bevorzugter 1 : 0.412 bis 1 : 1.546.

Es versteht sich von selbst, dass sich der Ausdruck "Summe aus der Masse des MHHPSA, der Masse des MNA, der Masse des MTHPA, der Masse des MFD, der Masse des HFDPA, der Masse des DMDF", wenn man ihn mit "$\Sigma_{Mono}$" abkürzt, mathematisch als

$$\Sigma_{Mono} = m_{MHHPSA} + m_{MNA} + m_{MTHPA} + m_{MFD} + m_{HFDPA} + m_{DMDF}$$

darstellen lässt, wobei

$m_{MHHPSA}$ = Masse des MHHPSA in Gramm;

$m_{MNA}$ = Masse des MNA in Gramm;

$M_{MTHPA}$ = Masse des MTHPA in Gramm;

$m_{MFD}$ = Masse des MFD in Gramm;

$m_{HFDPA}$ = Masse des HFDPA in Gramm;

$m_{DMDF}$ = Masse des DMDF in Gramm.

Das Einhalten dieser bevorzugten Massenverhältnisse erlaubt es, eine gut handhabbare und lagerstabile Zusammensetzung zu erhalten. Dies liegt daran, dass bei Einhalten dieser Massenverhältnisse die erfindungsgemäße Zusammensetzung gut verarbeitet und eingesetzt werden kann, da sie nicht staubt. Andererseits wird das Absetzen der schwereren Teilchen in der Zusammensetzung verhindert, wodurch keine Inhomogenitäten entstehen. Wie aus den Experimenten ersichtlich, ist dies im Falle der erfindungsgemäßen Zusammensetzung über einen breiten Bereich der Massenverhältnisse des OPDA bezogen auf die Summe der Masse von MHHPSA, MNA, MTHPA, MFD, HFDPA und DMDF in der Zusammensetzung möglich. Es wurde im Übrigen beobachtet, dass eine Mischung aus OPDA und MTHPA, verglichen mit einer Mischung aus OPDA und MNA, über einen breiteren Bereich an möglichen Massenverhältnissen diese vorteilhaften Eigenschaften zeigt. Dies war zusätzlich völlig überraschend.

Die eingesetzte Menge des OTA in der erfindungsgemäßen Zusammensetzung ist nicht besonders beschränkt. In einer besonders vorteilhaften Ausführungsform beträgt die Masse des OTA in der Zusammensetzung 0.01 bis 16.9 % der Summe aus der Masse des OPDA, der Masse des MHHPSA, der Masse des MNA, der Masse des MTHPA, der Masse des MFD, der Masse des HFDPA, der Masse des DMDF in der Zusammensetzung. Bevorzugt beträgt die Masse des OTA in der Zusammensetzung 0.025 bis 10 % der Summe aus der Masse des OPDA, der Masse des MHHPSA, der Masse des MNA, der Masse des MTHPA, der Masse des MFD, der Masse des HFDPA, der Masse des DMDF in der Zusammensetzung. Noch bevorzugter beträgt die Masse des OTA in der Zusammensetzung 0.05 bis 5 % der Summe aus der Masse des OPDA, der Masse des MHHPSA, der Masse des MNA, der Masse des MTHPA, der Masse

des MFD, der Masse des HFDPA, der Masse des DMDF in der Zusammensetzung. Am bevorzugtesten beträgt die Masse des OTA in der Zusammensetzung 0.05 bis 3 % der Summe aus der Masse des OPDA, der Masse des MHHPSA, der Masse des MNA, der Masse des MTHPA, der Masse des MFD, der Masse des HFDPA, der Masse des DMDF in der Zusammensetzung.

Der Ausdruck "Summe aus der Masse des OPDA, der Masse des MHHPSA, der Masse des MNA, der Masse des MTHPA, der Masse des MFD, der Masse des HFDPA, der Masse des DMDF" lässt sich, wenn man ihn mit "$\Sigma_{Mono + OPDA}$" abkürzt, mathematisch als

$$\Sigma_{Mono+ OPDA} = m_{OPDA} + \Sigma_{Mono}$$

darstellen, wobei $m_{OPDA}$ die Masse des OPDA in Gramm angibt und $\Sigma_{Mono}$ die oben genannte Bedeutung hat.

Die erfindungsgemäße Zusammensetzung wird insbesondere in Härtersystemen, bevorzugt solchen für Epoxidharze, eingesetzt.

Die Erfindung betrifft somit in einem weiteren Aspekt ein Härtersystem für Epoxidharze, das die erfindungsgemäße Zusammensetzung umfasst.

Es versteht sich von selbst, dass das erfindungsgemäße Härtersystem außer dem OPDA, dem OTA, dem MHHPSA, dem MNA, dem MTHPA, dem MFD, dem HFDPA, dem DMDF in der erfindungsgemäßen Zusammensetzung nicht noch mehr OPDA, OTA, MHHPSA, MNA, MTHPA, MFD, HFDPA, DMDF enthält.

Das erfindungsgemäße Härtersystem weist die erfindungsgemäße Zusammensetzung mit einem Massenanteil von 0.10 - 1.0, bevorzugt 0.20 - 0.999, noch bevorzugter 0.40 - 0.90, am bevorzugtesten 0.50 - 0.85, jeweils auf die gesamte Masse des Härtersystems bezogen, auf.

Das erfindungsgemäße Härtersystem kann zusätzlich noch weitere Additive, wie beispielsweise Gleitmittel, Antiblockmittel, Trennmittel, Stabilisatoren, beispielsweise Antioxidantien, Lichtschutzmittel, Wärmestabilisatoren oder Schaumstabilisatoren,

Antistatika, leitfähige Zusatzstoffe, Flammschutzmittel, Pigmente, Schlagzähmodifier, Flexibilisatoren, Weichmacher, Haftvermittler, Füllstoffe, beispielsweise Ruß, Calciumcarbonat, Talkum, Silikate, Baumwollflocken, synthetische Polymere, Metallpulver, Graphit oder Glasfasern, Verstärkungsmaterialien, Treibmittel, Kicker, Nukleierungsmittel, antibakterielle Mittel oder Fungizide aufweisen. Als die genannten Additive können alle Stoffe verwendet werden, die dem Fachmann zur Herstellung von Epoxidharzsystemen als geeignete Additive bekannt sind.

Das erfindungsgemäße Härtersystem zeichnet sich dadurch aus, dass auf den Einsatz einer Aminverbindung oder eines Metallsalzes als Katalysator verzichtet werden kann. Es ist deshalb bevorzugt, dass das erfindungsgemäße Härtersystem keine solchen Katalysatoren aufweist. Natürlich kann das erfindungsgemäße Härtersystem nichts desto trotz auch in Kombination mit einem solchen Katalysator eingesetzt werden kann.

Im Sinne der Erfindung ist eine "Aminverbindung" ausgewählt aus der Gruppe bestehend aus Aminen, phenolischen Aminen und cycloaliphatischen oder aromatischen *N*-Heterocyclen.

"Amine" sind im Sinne der Erfindung $N^1$,$N^1$-Dimethylpropan-1,3-diamin (DMAPA), $N^1$,$N^1$,$N^3$,$N^3$-tetramethylpropan-1,3-diamin, $N^1$,$N^1$,$N^2$,$N^2$-Tetramethylethan-1,2-diamin, *N,N*-Dimethyl-1-benzylamin, *N,N*-Diethyl-1-benzylamin, Triethylamin, Tripropylamin, Diisopropylethylamin, 2-Dimethylaminoethanol oder 2-Diethylaminoethanol.

"Cycloaliphatische oder aromatische *N*-Heterocyclen" sind im Sinne der Erfindung Pyrrolidin, Piperidin, 1-Benzylpiperidin, Piperazin, 1,4-Dimethylpiperazin, 2,2,6,6-Tetramethylpiperidin, 2,2,6,6-Tetramethylpiperidin-4-amin, *N*-Alkyl-2,2,6,6-tetramethylpiperidin-4-amin, $N^1$,$N^1$-Dimethyl-$N^3$-(2,2,6,6-tetramethylpiperidin-4-yl)propan-1,3-diamin, 2,2,6,6-Tetramethylpiperidin-4-ol, 1,2,2,6,6-Pentamethylpiperidin-4-ol, 4-Alkoxy-2,2,6,6-tetramethylpiperidin, $N^1$,$N^6$-Bis(2,2,6,6-tetramethylpiperidin-4-yl)hexan-1,6-diamin,1H-Pyrrol, 1*H*-Imidazol, 1-Methyl-1*H*-imidazol (1MZ), 3-(2-Ethyl-4-methyl-1*H*-imidazol-1-yl)propannitril (2E4MZ-CN), 2-Ethyl-4-methyl-1*H*-imidazol (2E4MZ), 2-Methyl-1*H*-imidazol (2MZ), 2-Phenyl-1*H*-imidazol (2PZ), 1-Benzyl-2-methyl-1*H*-imidazol (1 B2MZ), 1-Benzyl-2-phenyl-1*H*-imidazol (1B2PZ), (4-Methyl-2-phenyl-1*H*-imidazol-5-yl)methanol (2P4MHZ), (2-Phenyl-1*H*-imidazol-4,5-diyl)dimethanol (2PHZ), 6-(2-(2-Methyl-1*H*-imidazol-1-yl)ethyl)-1,3,5-triazin-2,4-diamin (2MZ-A), 2,3-Dihydro-1*H*-benzo[d]pyrrolo[1,2-a]imidazol (TBZ), Pyridin, 2-Methylpyridin, 3-Methylpyridin, 4-Methylpyridin, 2,6-Dimethylpyridin und 1,3-Dialkyl-1*H*-imidazol-3-iumsalze, insbesondere Carboxylate, Halogenide, Sulfonate, Nitrate, Sulfate oder Hydrogensulfate.

"Phenolische Amine" sind im Sinne der Erfindung 4-Dimethylaminomethylphenol, 2,6-Di-*tert*-butyl-4-dimethylaminomethylphenol (Ionol® 103), 2,4,6-Trisdimethylaminomethylphenol oder 2,4-Bisdimethylaminomethyl-6-methylphenol.

"Metallsalze" sind im Sinne der Erfindung Zink(II)-acetylacetonat, 1-Methylimidazolium-zink(II)-acetylacetonat [(1 MZ)Zn(acac)$_2$], Bis(1-methylimidazolium)-eisen(II)-acetylacetonat [(1MZ)$_2$(Fe(acac)$_2$], Zinnoctanoat oder Bortrifluoridkomplexe, insbesondere Etherate oder Komplexe mit Ethylamin.

Ebenfalls Gegenstand der vorliegenden Erfindung ist die Verwendung von Härtersystemen gemäß der vorliegenden Erfindung zur Härtung von Epoxidharzsystemen. Weiterhin sind Gegenstand der vorliegenden Erfindung Epoxidharzsysteme, umfassend mindestens ein Epoxidharz und mindestens ein Härtersystem gemäß der vorliegenden Erfindung. Das erfindungsgemäße Härtersystem hat den Vorteil, dass es als solches in die Epoxidharze eingearbeitet werden kann, ohne dass ein Zusatz weiterer Hilfsstoffe, insbesondere Lösungsmittel, erforderlich ist. Vorzugsweise enthält das erfindungsgemäße Epoxidharzsystem daher keine Lösungsmittel. Ein weiterer Vorteil der vorliegenden Erfindung liegt in der Möglichkeit, unterhalb des Schmelzpunkts des OPDA eine Aushärtung zu erreichen und dabei, bei Wahl geeigneter Härtungszyklen, zu ausgehärteten, wärmeformstabilen Systemen mit einer hohen Glastemperatur, insbesondere oberhalb von 200 °C zu gelangen.

Grundsätzlich gibt es keine Einschränkungen hinsichtlich der einzusetzenden Epoxidharze, das heißt es können auch Mischungen unterschiedlicher Epoxidharze vorliegen. Vorzugsweise liegt mindestens ein Epoxidharz mit mindestens 2 Epoxy-Gruppen pro Monomer vor. Das genannte Epoxidharz mit mindestens 2 Epoxy-Gruppen pro Monomer kann allein oder in Mischung mit weiteren Epoxidharzen eingesetzt werden.

Insbesondere bevorzugt liegen in dem erfindungsgemäßen Epoxidharzsystem keine aminischen Epoxidharze vor, wie sie beispielsweise in EP 0181337 oder EP 1091992 beschrieben sind.

Beispiele für geeignete Epoxidharze sind Epoxidharze vom Glycidylether-Typ, die aus Bisphenol A oder Bisphenol F und Epihalogenhydrinen synthetisiert werden können; Epoxidharze vom Glycidylester-Typ, die aus Phthalsäure und Epihalogenhydrinen synthetisiert werden können; alicyclische Epoxidharze, die durch Epoxidierung von alicyclischen Dienen, wie Cyclopentadien oder Cyclohexadien, erhalten werden können; Epoxidierungsprodukte von ungesättigten Polymeren, wie Polybutadien und Polyisopren; und Polymere oder Copolymere von ungesättigten Monoepoxiden, wie Glycidylmethacrylat oder Allylglycidylether. Diese Aufzählung ist lediglich beschreibend. So ist es beispielsweise möglich, verschiedene, mehrwertige Phenole anstelle von Bisphenol A einzusetzen oder andere, mehrbasische Säuren anstelle der Phthalsäure zu verwenden.

Der Anteil des Härtersystems in der Mischung mit den Epoxidharzen ergibt sich in der Regel aus dem Verhältnis der Anzahl der Anhydrid-Gruppen im Härtersystem zu der Anzahl der Epoxy-Gruppen der eingesetzten Epoxidharze. Pro mol enthaltener Epoxy-Gruppe des eingesetzten Epoxidharzes werden 0.3 - 1 mol, besonders bevorzugt 0.5 - 0.8 mol, ganz besonders bevorzugt 0.55 - 0.75 mol Anhydrid-Gruppen eingesetzt.

Bei der Verwendung des erfindungsgemäßen Härtersystems zur Härtung von Epoxidharzen sind mehrere gleichwertige Ausführungsformen denkbar.

In einer Ausführungsform der erfindungsgemäßen Erfindung wird zunächst das oben beschriebene Härtersystem hergestellt und anschließend mit mindestens einem Epoxidharz gemischt. Bevorzugt ist es bei dieser Ausführungsform, dass OPDA, OTA und mindestens eine Monoanhydridverbindung ausgewählt aus der Gruppe bestehend aus MHHPSA, MNA, MTHPA, MFD, HFDPA, DMDF in den oben beschriebenen Gewichtsanteilen vorliegen.

Ein wesentlicher Vorteil dieser Ausführungsform liegt darin, dass der Anwender nur das Epoxidharz und das Härtersystem bei der Anwendung im Sinne eines ZweiKomponentensystems zusammen bringen muss. Eine getrennte Aufbewahrung der Einzelkomponenten des Härtersystems ist nicht erforderlich, was zu einer vereinfachten Anwendbarkeit führt.

In einer weiteren Ausführungsform der vorliegenden Erfindung kann zunächst nur eine Mischung des mindestens einen Epoxidharzes mit OPDA und der mindestens einen Monoanhydridverbindung ausgewählt aus der Gruppe bestehend aus MHHPSA, MNA, MTHPA, MFD, HFDPA, DMDF vorliegen, bevorzugt in den oben beschriebenen Gewichtsanteilen, zu der anschließend das OTA, bevorzugt in den oben beschriebenen Gewichtsanteilen separat zugegeben wird.

In Summe liegt dann nach Zugabe des OTA zu dem Epoxidharzsystem ebenfalls wieder die erfindungsgemäße Kombination des Härtersystems vor.

Hierzu wird zunächst OPDA mit mindestens einer Monoanhydridverbindung ausgewählt aus der Gruppe bestehend aus MHHPSA, MNA, MTHPA, MFD, HFDPA, DMDF vermischt. Diese Mischung wird dann dem mindestens einen Epoxidharz zugegeben. Zur eigentlichen Härtung wird dann OTA zugegeben und die Härtung durchgeführt. Für die Erreichung des erfindungswesentlichen Vorteils kommt es nur darauf an, dass bei der eigentlichen Aushärtung der Epoxidharze das erfindungsgemäße Epoxidharz vorliegt. Dem Anwender wird damit die Möglichkeit gegeben, die Vorteile des erfindungsgemäßen Härtersystems zu nutzen, aber gleichzeitig zusätzlich Freiheit hinsichtlich der Reihenfolge der Zugabe der Einzelkomponenten zu erlangen.

Somit sind Verfahren zur Härtung von Epoxidharzsystemen ebenfalls Aspekte der Erfindung.

In einem ersten Aspekt ist dies eine Verfahren zur Härtung von Epoxidharzen, wobei

a. in einem ersten Schritt mindestens ein Epoxidharz mit

   i. OPDA; und
   ii. mindestens einer Monoanhydridverbindung ausgewählt aus der Gruppe bestehend aus MHHPSA, MNA, MTHPA, MFD, HFDPA, DMDF

gemischt wird;

b. in einem zweiten Schritt OTA zugegeben wird; und

c. in einem dritten Schritt das Epoxidharz bei einer Temperatur von mindestens 25 °C ausgehärtet wird.

Bevorzugt ist die Monoanhydridverbindung in dem Verfahren des ersten Aspekts der Erfindung in Schritt a.ii. ausgewählt aus der Gruppe bestehend aus MHHPSA, MTHPA, MNA. Noch bevorzugter ist die Monoanhydridverbindung in dem Verfahren des ersten Aspekts der Erfindung in Schritt a.ii. ausgewählt aus der Gruppe bestehend aus MTHPA, MNA. Am bevorzugtesten ist die Monoanhydridverbindung in dem Verfahren des ersten Aspekts der Erfindung in Schritt a.ii. MTHPA.

In einer vorteilhaften Ausführungsform des Verfahrens des ersten Aspekts der vorliegenden Erfindung beträgt das Verhältnis der Masse von eingesetztem OPDA zu der Summe aus der Masse des eingesetzten MHHPSA, der Masse des eingesetzten MNA, der Masse des eingesetzten MTHPA, der Masse des eingesetzten MFD, der Masse des eingesetzten HFDPA, der Masse des eingesetzten DMDF 1: 0.250 bis 1: 1.900, bevorzugt 1 : 0.309 bis 1 : 1.856, bevorzugter 1 : 0.412 bis 1 : 1.546.

In einer besonders vorteilhaften Ausführungsform des Verfahrens des ersten Aspekts der Erfindung beträgt die Masse des im Verfahren eingesetzten OTA 0.01 % bis 16.9 % der Summe aus der Masse des eingesetzten OPDA, der Masse des eingesetzten MHHPSA, der Masse des eingesetzten MNA, der Masse des eingesetzten MTHPA, der Masse des eingesetzten MFD, der Masse des eingesetzten HFDPA, der Masse des eingesetzten DMDF.

In einer bevorzugten Ausführungsform des Verfahrens des ersten Aspekts der Erfindung beträgt die Masse des im Verfahren eingesetzten OTA 0.025 % bis 10 % der Summe aus der Masse des eingesetzten OPDA, der Masse des eingesetzten MHHPSA, der Masse des eingesetzten MNA, der Masse des eingesetzten MTHPA, der Masse des eingesetzten MFD, der Masse des eingesetzten HFDPA, der Masse des eingesetzten DMDF.

In einer bevorzugteren Ausführungsform des Verfahrens des ersten Aspekts der Erfindung beträgt die Masse des im Verfahren eingesetzten OTA 0.05 % bis 5 % der Summe aus der Masse des eingesetzten OPDA, der Masse des eingesetzten MHHPSA, der Masse des eingesetzten MNA, der Masse des eingesetzten MTHPA, der Masse des eingesetzten MFD, der Masse des eingesetzten HFDPA, der Masse des eingesetzten DMDF.

In einer noch bevorzugteren Ausführungsform des Verfahrens des ersten Aspekts der Erfindung beträgt die Masse des im Verfahren eingesetzten OTA 0.05 % bis 3 % der Summe aus der Masse des eingesetzten OPDA, der Masse des eingesetzten MHHPSA, der Masse des eingesetzten MNA, der Masse des eingesetzten MTHPA, der Masse des eingesetzten MFD, der Masse des eingesetzten HFDPA, der Masse des eingesetzten DMDF.

In einem zweiten Aspekt ist dies eine Verfahren zur Härtung von Epoxidharzen, wobei

a. in einem ersten Schritt

i. OPDA; und
ii. OTA; und
iii. mindestens eine Monoanhydridverbindung ausgewählt aus der Gruppe bestehend aus MHHPSA, MNA, MTHPA, MFD, HFDPA, DMDF

gemischt werden;

b. in einem zweiten Schritt mindestens ein Epoxidharz zugegeben wird; und

c. in einem dritten Schritt das Epoxidharz bei einer Temperatur von mindestens 25 °C ausgehärtet wird.

Bevorzugt ist die Monoanhydridverbindung in dem Verfahren des zweiten Aspekts der Erfindung in Schritt a.iii. ausgewählt aus der Gruppe bestehend aus MHHPSA, MTHPA, MNA. Noch bevorzugter ist die Monoanhydridverbindung in dem Verfahren des zweiten Aspekts der Erfindung in Schritt a.iii. ausgewählt aus der Gruppe bestehend aus MTHPA, MNA. Am bevorzugtesten ist die Monoanhydridverbindung in dem Verfahren des zweiten Aspekts der Erfindung in Schritt a.iii. MTHPA.

In einer vorteilhaften Ausführungsform des Verfahrens des zweiten Aspekts der vorliegenden Erfindung beträgt das Verhältnis der Masse von eingesetztem OPDA zu der Summe aus der Masse des eingesetzten MHHPSA, der Masse des eingesetzten MNA, der Masse des eingesetzten MTHPA, der Masse des eingesetzten MFD, der Masse des eingesetzten HFDPA, der Masse des eingesetzten DMDF 1: 0.250 bis 1: 1.900, bevorzugt 1 : 0.309 bis 1 : 1.856, bevorzugter 1 : 0.412 bis 1 : 1.546.

In einer besonders vorteilhaften Ausführungsform des Verfahrens des zweiten Aspekts der Erfindung beträgt

die Masse des im Verfahren eingesetzten OTA 0.01 % bis 16.9 % der Summe aus der Masse des eingesetzten OPDA, der Masse des eingesetzten MHHPSA, der Masse des eingesetzten MNA, der Masse des eingesetzten MTHPA, der Masse des eingesetzten MFD, der Masse des eingesetzten HFDPA, der Masse des eingesetzten DMDF.

In einer bevorzugten Ausführungsform des Verfahrens des zweiten Aspekts der Erfindung beträgt die Masse des im Verfahren eingesetzten OTA 0.025 % bis 10 % der Summe aus der Masse des eingesetzten OPDA, der Masse des eingesetzten MHHPSA, der Masse des eingesetzten MNA, der Masse des eingesetzten MTHPA, der Masse des eingesetzten MFD, der Masse des eingesetzten HFDPA, der Masse des eingesetzten DMDF.

In einer bevorzugteren Ausführungsform des Verfahrens des zweiten Aspekts der Erfindung beträgt die Masse des im Verfahren eingesetzten OTA 0.05 % bis 5 % der Summe aus der Masse des eingesetzten OPDA, der Masse des eingesetzten MHHPSA, der Masse des eingesetzten MNA, der Masse des eingesetzten MTHPA, der Masse des eingesetzten MFD, der Masse des eingesetzten HFDPA, der Masse des eingesetzten DMDF.

In einer noch bevorzugteren Ausführungsform des Verfahrens des zweiten Aspekts der Erfindung beträgt die Masse des im Verfahren eingesetzten OTA 0.05 % bis 3 % der Summe aus der Masse des eingesetzten OPDA, der Masse des eingesetzten MHHPSA, der Masse des eingesetzten MNA, der Masse des eingesetzten MTHPA, der Masse des eingesetzten MFD, der Masse des eingesetzten HFDPA, der Masse des eingesetzten DMDF.

In den erfindungsgemäßen Verfahren zur Härtung von Epoxidharzsystemen gemäß dem obigen ersten und zweiten Aspekt erfolgt die Aushärtung bei einer Temperatur von mindestens 25 °C, insbesondere bei einer Temperatur von mindestens 50 °C. Die Aushärtung kann etwa bei einer Temperatur in Bereich von 25 °C bis unterhalb der Schmelztemperatur des OPDA erfolgt, insbesondere bei einer Temperatur im Bereich von 25 °C bis 200 °C, bevorzugt bei einer Temperatur im Bereich von 25 °C bis 180 °C. Die Schmelztemperatur des OPDA liegt im Bereich von 225 °C bis 229 °C.

Die vorliegende Erfindung wird anhand der nachfolgenden Beispiele illustriert, ohne auf diese beschränkt zu sein.

## Beispiele

### 1. Beispiele 1 (erfindungsgemäß) und 2 (Vergleichsbeispiel): Bestimmung der Gelzeit bei 170 ° C nach DIN EN 16 945, Blatt 1

Beispiel 1: In einem 100 ml Becherglas wurden bei Raumtemperatur 7.1 g OPDA, 2.9 g OTA und 10.0 g MNA (entspricht einer Masse des OTA von 16.9 % der Summe der Masse des OPDA und der Masse des MNA) mit einander vermengt. Anschließend wurden mit einem Holzspatel 10 g des cycloaliphatischen Epoxidharzes CY179 mit folgender Strukturformel

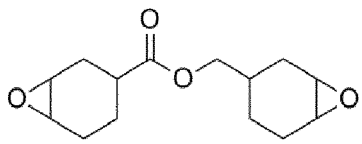

CY179

eingearbeitet, so dass eine homogene Masse entsteht.

10 g der so erhaltenen Mischung wurden in ein Reagenzglas umgefüllt, anschließend wurde nach DIN EN 16945, Blatt 1 die Gelzeit bei 170°C bestimmt. Die Gelzeit betrug 27.33 min.

Beispiel 2: Zum Vergleich wurde der Versuch ohne OTA wiederholt. Dazu wurden in einem 100 ml Becherglas bei Raumtemperatur 10.0 g OPDA und 10.0 g MNA mit einander vermengt. Anschließend wurden mit einem Holzspatel 10 g des cycloaliphatischen Epoxidharzes CY179 eingearbeitet, so dass eine homogene Masse entsteht.

10 g der so erhaltenen Mischung wurden in ein Reagenzglas umgefüllt, anschließend wurde nach DIN EN 16945, Blatt 1 die Gelzeit bei 170°C bestimmt. Die Gelzeit betrug 68.42 min.

Dies zeigt, dass schon bei sehr kleinen Anteilen an OTA in der Mischung eine deutliche Verringerung der Gelzeit beobachtet wurde. Dies ist auf die beschleunigende und völlig überraschende Eigenschaft des OTA zurückzuführen.

### 2. Beispiele 3 - 24 (erfindungsgemäß): Formulierung

In einem 100 ml- Becherglas wurden 9.7 g OPDA und 0.3 g OTA vorgelegt. Darauf wurde schrittweise MTHPA oder MNA entsprechend der in der Tabelle 1 gezeigten Mengen zugegeben und etwa eine Minute lang zu einer homogenen Masse bei 23 °C verrührt. Daraufhin wurde die Konsistenz visuell überprüft. Die folgende Tabelle 1 gibt in der vorletzten Spalte die Konsistenz der erhaltenen Mischung für den Fall, in dem als Monoanhydridverbindung MTHPA eingesetzt wurde (Spalte "MTHPA") und in der letzten Spalte die Konsistenz für den Fall, in dem als Monoanhydridverbindung MNA eingesetzt wurde (Spalte "MNA") an. "Rieselfähig" bedeutet dabei, dass keine Paste erhalten wurde, sondern die Mischung pulverförmig vorlag. "Pastös" bedeutet, dass die homogene Masse streichfest vorlag und auch über einen langen Zeitraum, das heißt mehr als eine Stunde, homogen blieb, ohne dass sich der suspendierte Feststoffanteil bestehend aus OTA und OPDA absetzte. "Instabil" bedeutet, dass die erhaltene Mischung zwar zunächst homogen war, sich aber nach weniger als einer Stunde der suspendierte Feststoffanteil bestehend aus OTA und OPDA absetzte. Die linke Spalte in der Tabelle 1 gibt die Nummer des jeweiligen Beispiels an. Ein Beispiel besteht jeweils aus zwei Versuchen, wobei in dem ersten die entsprechende Menge MTHPA und in dem zweiten die entsprechende Menge an MNA als Monoanhydridverbindung eingesetzt wurde.

Aus der Tabelle 1 geht hervor, dass sich im Falle, dass MTHPA als Monoanhydrid in Kombination mit OPDA eingesetzt wird, eine vorteilhafte pastöse Struktur über einen sehr breiten Mischungsbereich ergibt. So wurde die nachteilige rieselfähige Konsistenz erst bei einem Mischungsverhältnis von OPDA : MTHPA von 1 : 0.206 oder für noch kleinere Anteile von MTHPA gefunden, während eine Instabilität erst bei einem Anteil von 1 : 1.959 oder für noch höhere Anteile von MTHPA festgestellt wurde. Der entsprechende Wertebereich bei MNA ist viel schmaler: So wurde die nachteilige rieselfähige Konsistenz noch bei einem Mischungsverhältnis von OPDA : MNA von 1 : 0.361 oder für noch kleinere Anteile von MNA gefunden, während eine Instabilität schon bei einem Anteil von 1 : 1.649 oder für noch höhere Anteile von MNA festgestellt wurde. Dieser breite Bereich, in dem MTHPA mit OPDA gemischt werden konnte und eine gut verarbeitbare Struktur ergibt, war gegenüber den Ergebnissen im Falle der Kombination von OPDA und MNA völlig überraschend.

**Tabelle 1**

| | OTA [g] | OPDA [g] | Monoanhydrid [g] | Mischungsverhältnis OPDA | Monoanhydrid | MTHPA | MNA |
|---|---|---|---|---|---|---|---|
| 3 | 0.3 | 9.7 | 1.0 | 1.000 | 0.103 | Rieselfähig | Rieselfähig |
| 4 | 0.3 | 9.7 | 2.0 | 1.000 | 0.206 | Rieselfähig | Rieselfähig |
| 5 | 0.3 | 9.7 | 3.0 | 1.000 | 0.309 | Pastös | Rieselfähig |
| 6 | 0.3 | 9.7 | 3.5 | 1.000 | 0.361 | Pastös | Rieselfähig |
| 7 | 0.3 | 9.7 | 4.0 | 1.000 | 0.412 | Pastös | Pastös |
| 8 | 0.3 | 9.7 | 5.0 | 1.000 | 0.515 | Pastös | Pastös |
| 9 | 0.3 | 9.7 | 6.0 | 1.000 | 0.619 | Pastös | Pastös |
| 10 | 0.3 | 9.7 | 7.0 | 1.000 | 0.722 | Pastös | Pastös |
| 11 | 0.3 | 9.7 | 8.0 | 1.000 | 0.825 | Pastös | Pastös |
| 12 | 0.3 | 9.7 | 9.0 | 1.000 | 0.928 | Pastös | Pastös |
| 13 | 0.3 | 9.7 | 10.0 | 1.000 | 1.031 | Pastös | Pastös |
| 14 | 0.3 | 9.7 | 11.0 | 1.000 | 1.134 | Pastös | Pastös |
| 15 | 0.3 | 9.7 | 12.0 | 1.000 | 1.237 | Pastös | Pastös |
| 16 | 0.3 | 9.7 | 13.0 | 1.000 | 1.340 | Pastös | Pastös |
| 17 | 0.3 | 9.7 | 14.0 | 1.000 | 1.443 | Pastös | Pastös |
| 18 | 0.3 | 9.7 | 15.0 | 1.000 | 1.546 | Pastös | Pastös |
| 19 | 0.3 | 9.7 | 16.0 | 1.000 | 1.649 | Pastös | Instabil |
| 20 | 0.3 | 9.7 | 17.0 | 1.000 | 1.753 | Pastös | Instabil |
| 21 | 0.3 | 9.7 | 18.0 | 1.000 | 1.856 | Pastös | Instabil |
| 22 | 0.3 | 9.7 | 19.0 | 1.000 | 1.959 | Instabil | Instabil |
| 23 | 0.3 | 9.7 | 19.5 | 1.000 | 2.010 | Instabil | Instabil |
| 24 | 0.3 | 9.7 | 20.0 | 1.000 | 2.062 | Instabil | Instabil |

## Patentansprüche

1. Zusammensetzung umfassend

   a. 5,5'-Oxybis(isobenzofuran-1,3-dion);

b. 4,4'-Oxybis(*ortho*-phthalsäure); und
c. mindestens eine Monoanhydridverbindung ausgewählt aus der Gruppe bestehend aus Methylhexahydroisobenzofuran-1,3-dion, 5-Methyl-3a,4,7,7a-tetrahydro-4,7-methanoisobenzofuran-1,3-dion, 5-Methyl-3a,4,7,7a-tetrahydroisobenzofuran-1,3-dion, 3-Methylfuran-2,5-dion, 3,3,4,4,5,5-Hexafluorodihydro-2*H*-pyran-2,6(3*H*)-dion, 3,3-Dimethyldihydrofuran-2,5-dion.

**2.** Zusammensetzung nach Anspruch 1, **dadurch gekennzeichnet, dass** die Monoanhydridverbindung ausgewählt ist aus der Gruppe bestehend aus Methylhexahydroisobenzofuran-1,3-dion, 5-Methyl-3a,4,7,7a-tetrahydroisobenzofuran-1,3-dion, 5-Methyl-3a,4,7,7a-tetrahydro-4,7-methanoisobenzofuran-1,3-dion.

**3.** Zusammensetzung nach Anspruch 1 oder 2, **dadurch gekennzeichnet, dass** die Monoanhydridverbindung ausgewählt ist aus der Gruppe bestehend aus 5-Methyl-3a,4,7,7a-tetrahydroisobenzofuran-1,3-dion, 5-Methyl-3a,4,7,7a-tetrahydro-4,7-methanoisobenzofuran-1,3-dion.

**4.** Zusammensetzung nach einem oder mehreren der Ansprüche 1 bis 3, **dadurch gekennzeichnet, dass** die Monoanhydridverbindung 5-Methyl-3a,4,7,7a-tetrahydroisobenzofuran-1,3-dion ist.

**5.** Zusammensetzung nach einem oder mehreren der Ansprüche 1 bis 4, wobei das Verhältnis der Masse von 5,5'-Oxybis(isobenzofuran-1,3-dion) in der Zusammensetzung zur Summe aus der Masse des Methylhexahydroisobenzofuran-1,3-dions, der Masse des 5-Methyl-3a,4,7,7a-tetrahydro-4,7-methanoisobenzofuran-1,3-dions, der Masse des 5-Methyl-3a,4,7,7a-tetrahydroisobenzofuran-1,3-dions, der Masse des 3-Methylfuran-2,5-dions, der Masse des 3,3,4,4,5,5-Hexafluorodihydro-2*H*-pyran-2,6(3*H*)-dions, der Masse des 3,3-Dimethyldihydrofuran-2,5-dions in der Zusammensetzung 1 : 0.250 bis 1 : 1.900 beträgt.

**6.** Zusammensetzung nach einem oder mehreren der Ansprüche 1 bis 5, wobei die Masse des 4,4'-Oxybis(*ortho*-phthalsäure) in der Zusammensetzung 0.01 bis 16.9 % der Summe aus der Masse des 5,5'-Oxybis(isobenzofuran-1,3-dion), der Masse des Methylhexahydroisobenzofuran-1,3-dions, der Masse des 5-Methyl-3a,4,7,7a-tetrahydro-4,7-methanoisobenzofuran-1,3-dions, der Masse des 5-Methyl-3a,4,7,7a-tetrahydroisobenzofuran-1,3-dions, der Masse des 3-Methylfuran-2,5-dions, der Masse des 3,3,4,4,5,5-Hexafluorodihydro-2*H*-pyran-2,6(3*H*)-dions, der Masse des 3,3-Dimethyldihydrofuran-2,5-dions in der Zusammensetzung beträgt.

**7.** Härtersystem für Epoxidharze, umfassend die Zusammensetzung nach einem oder mehreren der Ansprüche 1 bis 6 mit einem Massenanteil von 0.10 bis 1.0, bezogen auf die gesamte Masse des Härtersystems.

**8.** Härtersystem nach Anspruch 7, **dadurch gekennzeichnet, dass** es keine Aminverbindung aufweist.

**9.** Härtersystem nach Anspruch 7 oder 8, **dadurch gekennzeichnet, dass** es kein Metallsalz aufweist.

**10.** Verwendung eines Härtersystems gemäß einem oder mehreren der Ansprüche 7 bis 9 zur Härtung von Epoxidharzen.

**11.** Epoxidharzsystem umfassend ein Epoxidharz und mindestens ein Härtersystem gemäß einem oder mehreren der Ansprüche 7 bis 9.

**12.** Verfahren zur Härtung von Epoxidharzen, wobei

a. in einem ersten Schritt mindestens ein Epoxidharz mit

i. 5,5'-Oxybis(isobenzofuran-1,3-dion); und
ii. mindestens einer Monoanhydridverbindung ausgewählt aus der Gruppe bestehend aus Methylhexahydroisobenzofuran-1,3-dion, 5-Methyl-3a,4,7,7a-tetrahydro-4,7-methanoisobenzofuran-1,3-dion, 5-Methyl-3a,4,7,7a-tetrahydroisobenzofuran-1,3-dion, 3-Methylfuran-2,5-dion, 3,3,4,4,5,5-Hexafluorodihydro-2*H*-pyran-2,6(3*H*)-dion, 3,3-Dimethyldihydrofuran-2,5-dion gemischt wird;

b. in einem zweiten Schritt 4,4'-Oxybis(*ortho*-phthalsäure) zugegeben wird; und
c. in einem dritten Schritt das Epoxidharz bei einer Temperatur von mindestens 25 °C ausgehärtet wird.

**13.** Verfahren zur Härtung von Epoxidharzen, wobei

a. in einem ersten Schritt

i. 5,5'-Oxybis(isobenzofuran-1,3-dion); und
ii. 4,4'-Oxybis(*ortho*-phthalsäure); und
iii. mindestens eine Monoanhydridverbindung ausgewählt aus der Gruppe bestehend aus Methylhexahydroisobenzofuran-1,3-dion, 5-Methyl-3a,4,7,7a-tetrahydro-4,7-methanoisobenzofuran-1,3-dion, 5-Methyl-3a,4,7,7a-tetrahydroisobenzofuran-1,3-dion, 3-Methylfuran-2,5-dion, 3,3,4,4,5,5-Hexafluorodihydro-2*H*-pyran-2,6(3*H*)-dion, 3,3-Dimethyldihydrofuran-2,5-dion gemischt werden;

b. in einem zweiten Schritt mindestens ein Epoxidharz zugegeben wird; und
c. in einem dritten Schritt das Epoxidharz bei einer Temperatur von mindestens 25 °C ausgehärtet wird.

**14.** Verfahren gemäß Anspruch 12 oder 13, **dadurch gekennzeichnet, dass** die Aushärtung bei einer Temperatur im Bereich von 25 °C bis unterhalb der Schmelztemperatur des 5,5'-Oxybis(isobenzofuran-1,3-dion) erfolgt.

Europäisches Patentamt
European Patent Office
Office européen des brevets

# EUROPÄISCHER RECHERCHENBERICHT

Nummer der Anmeldung
EP 14 19 6246

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A,P | EP 2 679 623 A1 (EVONIK INDUSTRIES AG [DE]) 1. Januar 2014 (2014-01-01)<br>* das ganze Dokument *<br>----- | 1-14 | INV.<br>C08K5/00<br>C08G59/42<br>C07C51/00<br>C07D307/78 |

RECHERCHIERTE SACHGEBIETE (IPC)
C08K
C08G
C07D
C07C

Der vorliegende Recherchenbericht wurde für alle Patentansprüche erstellt

1

| Recherchenort | Abschlußdatum der Recherche | Prüfer |
|---|---|---|
| Den Haag | 28. April 2015 | Siemens, Thomas |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..........................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 14 19 6246

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

28-04-2015

| Im Recherchenbericht angeführtes Patentdokument | Datum der Veröffentlichung | Mitglied(er) der Patentfamilie | Datum der Veröffentlichung |
|---|---|---|---|
| EP 2679623 A1 | 01-01-2014 | CN 103524715 A | 22-01-2014 |
| | | DE 102012211323 A1 | 02-01-2014 |
| | | EP 2679623 A1 | 01-01-2014 |
| | | EP 2749589 A1 | 02-07-2014 |
| | | JP 2014009360 A | 20-01-2014 |
| | | KR 20140002551 A | 08-01-2014 |
| | | TW 201418317 A | 16-05-2014 |
| | | US 2014005344 A1 | 02-01-2014 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4002599 A
- DE 2837726
- US 3989573 A
- EP 0181337 A
- EP 1091992 A


### In der Beschreibung aufgeführte Nicht-Patentliteratur


- Methoden der Organischen Chemie. **HOUBEN-WEYL.** Makromolekulare Stoffe. Georg Thieme Verlag, 1987, vol. E20, 1959